(19) Europäisches Patentamt / European Patent Office / Office européen des brevets

(11) **EP 3 544 537 B1**

(12) **EUROPEAN PATENT SPECIFICATION**

(45) Date of publication and mention
of the grant of the patent:
**19.03.2025 Bulletin 2025/12**

(21) Application number: **17873801.9**

(22) Date of filing: **21.11.2017**

(51) International Patent Classification (IPC):
*A61B 34/20* (2016.01)   *A61B 90/00* (2016.01)
*A61B 6/03* (2006.01)   *A61B 5/055* (2006.01)

(52) Cooperative Patent Classification (CPC):
**A61B 6/032; A61B 6/12; A61B 6/463;
A61B 6/5247; A61B 8/0841; A61B 8/4263;
A61B 8/4416; A61B 90/36; A61B 90/37;**
A61B 5/055; A61B 2017/3413; A61B 2034/2055;
A61B 2034/2063; A61B 2090/364; A61B 2090/365;
(Cont.)

(86) International application number:
**PCT/US2017/062883**

(87) International publication number:
**WO 2018/098198 (31.05.2018 Gazette 2018/22)**

(54) **SYSTEM AND METHODS FOR INTERVENTIONAL IMAGE NAVIGATION AND IMAGE REGISTRATION REFINEMENT**

SYSTEM UND VERFAHREN ZUR INTERVENTIONELLEN NAVIGATION UND VERFEINERUNG VON BILDREGISTRIERUNG

SYSTÈME ET PROCÉDÉS DE NAVIGATION EN IMAGERIE INTERVENTIONNELLE ET D'AFFINEMENT D'ENREGISTREMENT D'IMAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.11.2016 US 201662426024 P**

(43) Date of publication of application:
**02.10.2019 Bulletin 2019/40**

(73) Proprietor: **Clear Guide Medical, Inc.
Baltimore, Maryland 21211 (US)**

(72) Inventors:
• **STOLKA, Philipp Jakob
85276 Pfaffenhofen an der Ilm (DE)**
• **BASAFA, Ehsan
Redwood City
CA 94065 (US)**

(74) Representative: **Lippert Stachow Patentanwälte
Rechtsanwälte
Partnerschaft mbB
Postfach 30 02 08
51412 Bergisch Gladbach (DE)**

(56) References cited:
EP-B1- 1 772 745     WO-A2-01/34051
WO-A2-2011/025943     US-A1- 2006 237 652
US-A1- 2007 083 117     US-A1- 2011 052 033
US-A1- 2012 071 757     US-A1- 2013 053 679
US-A1- 2016 104 287

• **MATHIAS MARKERT: "Manual registration of ultrasound with CT/planning data for hepatic surgery - PubMed", 31 December 2007 (2007-12-31), XP055730171, Retrieved from the Internet <URL:https://pubmed.ncbi.nlm.nih.gov/17377294/> [retrieved on 20200914]**

- CHA DONG IK ET AL: "A prospective comparison between auto-registration and manual registration of real-time ultrasound with MR images for percutaneous ablation or biopsy of hepatic lesions", ABDOMINAL RADIOLOGY, SPRINGER US, NEW YORK, vol. 42, no. 6, 13 February 2017 (2017-02-13), pages 1799 - 1808, XP036250412, ISSN: 2366-004X, [retrieved on 20170213], DOI: 10.1007/S00261-017-1075-X
- XU, S. ET AL.: "Real-time MRI-TRUS fusion for guidance of targeted prostate biopsies", COMPUTER AIDED SURGERY, vol. 13, no. 5, 2008, pages 255 - 264, XP009114007

(52) Cooperative Patent Classification (CPC): (Cont.)
A61B 2090/3762; A61B 2090/378; A61B 2090/3925;
A61B 2090/3937; A61B 2090/3966;
A61B 2090/3995

**Description**

**CROSS-REFERENCE TO RELATED APPLICATIONS**

The present patent application claims priority benefit from U.S. Provisional Patent Application No. 62/426,024 filed on November 23, 2016.

**BACKGROUND**

1. Technical Field

This disclosure generally relates to image systems for navigating surgical medical devices. More particularly, this disclosure relates to systems and methods for navigating interventional instrumentation, such as surgical needles, and refining registration between image modalities.

2. Discussion of Related Art

In the course of performing a surgical operation or intervention, a medical practitioner (e.g., a surgeon) may use various operating instruments to perform various operations such as needle biopsies, tumor ablations, catheter insertion, orthopedic interventions, etc. These instruments may use several types of image data to aid the medical practitioner or operator in inserting the instrument into a desired location within a patient's body. Typically, the medical practitioner inserts these instruments into a patient's body at very specific locations, orientations, and depths to reach predetermined target areas in order to perform an instrument-specific action or function, which may include tissue sampling, heating, cooling, liquid deposition, suction, or serving as a channel for other objects.

Medical navigation systems based on a variety of different tracking technologies (mechanical, infrared-optical, electromagnetic, etc.) have existed for a long time, and help with aligning patient, imaging data, targets, and instruments. However, the known medical navigation systems remain inaccurate, inconvenient, and ineffective in providing real-time data and guidance in inserting and moving interventional instruments.

Therefore, a need remains for improved systems and methods for interventional image navigation and image registration refinement. US 2007/083117 discloses a method of registering ultrasound image data of an object (3) and second image data (SD) of the object (3), wherein the second image data (SD) are three-dimensional (3-D) image data.

**SUMMARY OF THE DISCLOSURE**

The invention is defined by the appended claims. An aspect of the present disclosure is to provide a system for surgical image guidance. The system includes: an ultrasound imaging device capable of real-time imaging of a patient's body, tracking camera mounted to the ultrasound imagining device, a tracking system and an image processing system operatively connected to the ultrasound imaging device and the tracking system. The tracking system is configured to receive optical image data from the tracking camera and to determine a position of the ultrasound imaging device relative to the patient's body, based on a plurality of fiducial markers attached to the patient's body and visible in the optical image data. The image processing system is configured to: 1) receive a real-time ultrasound image of the patient from the ultrasound imaging device and a three-dimensional (3D) image dataset of the patient from another imaging device, said plurality of fiducial markers also visible in the 3D image dataset; 2) determine a position of each of the plurality of fiducial markers relative to the 3D image dataset; 3) perform a registration of the real-time ultrasound image with the 3D image dataset, based on the position of the ultrasound imaging device relative to the patient's body and on the position of the fiducial markers relative to the 3D image dataset; 4) based on the registration of the real-time ultrasound image with the 3D image dataset, extract a spatially corresponding image from the 3D image dataset; 5) produce a composite image based on an overlay of the real-time image with the extracted image from the 3D image dataset; 6) provide a mode in which the real-time ultrasound image is free to move relative to the extracted image from the 3D image dataset; 7) refine the registration by receiving at least one additional real-time ultrasound image in which physical structures in the extracted image overlap with corresponding physical structures in the at least one additional real-time ultrasound image; and 8) provide automatic or manual means to enter a mode in which the real-time ultrasound image and the extracted image from the 3D image dataset are locked relative to each other to prevent relative movement therebetween. The system further comprises a display device configured to receive the composite image from the image processing system and display the composite image.

Another aspect of the present disclosure is to provide a method for surgical image guidance. The method includes receiving a real-time ultrasound image of a patient from an ultrasound imaging device, a three-dimensional (3D) image dataset of the patient from a another imaging device, and a position of the ultrasound imaging device relative to the patient's body, wherein a plurality of fiducial markers are visible in the 3D image dataset; determining a position of each of the plurality of fiducial markers relative to the 3D image dataset; performing a registration of the real-time ultrasound image with the 3D image dataset, based on the position of the ultrasound imaging device relative to the patient's body and on the position of the fiducial markers relative to the 3D image dataset; based on the registration of the real-time ultrasound image with the 3D image dataset, extracting a spatially corresponding image from the 3D image dataset; producing a composite image based on an overlay of the real-time image with the extracted image from the 3D image dataset; providing a mode in which the real-time ultrasound image is free to move relative to the extracted image from the 3D image dataset; refining the registration by receiving at least one additional real-time ultrasound image in which physical structures in the extracted image overlap with corresponding physical structures in the additional real-time ultrasound image; providing automatic or manual means to enter a mode in which the real-time ultrasound image and the extracted image from the 3D image dataset are locked relative to each other to prevent relative movement therebetween. The method further includes displaying the composite image on a display device.

## BRIEF DESCRIPTION OF THE DRAWINGS

The present disclosure, as well as the methods of operation and functions of the related elements of structure and the combination of parts and economies of manufacture, will become more apparent upon consideration of the following description and the appended claims with reference to the accompanying drawings, all of which form a part of this specification. Like reference numerals designate corresponding parts in the various figures. The drawings are for the purpose of illustration and description only and are not intended as a definition of the limits of the invention.

FIG. 1 depicts an example of an ultrasound system (Clear Guide SCENERGY system) having an ultrasound probe having mounted thereon a tracking device (e.g., one or more cameras) for tracking a position of the ultrasound probe on a patient's body;

FIG. 2 depicts the insertion of a needle into a patient's body by a health practitioner while using an ultrasound system (Clear Guide Medical "SCENERGY" system) having the one or more cameras to track the position of the needle relative to patient's body, the ultrasound system providing an image displaying information about a path of the needle insertion within the patient's body;

FIG. 3 shows a plurality of VisiMARKERs that are applied in an irregular fashion around the intervention area in a body of a patient;

FIG. 4 depicts a camera sweep wherein the camera captures a plurality of images including the VisiMARKERS;

FIG. 5 illustrates a position of the markers on the body of the patient, and shows images corresponding to one image modality (e.g., CT scan image modality) superposed thereon a track of a surgical device (e.g., a needle) being inserted into the body of the patient and another image modality (e.g., ultrasound scan image modality) superposed thereon a track of a surgical device (e.g., a needle) being inserted into the body of the patient;

FIG. 6A depicts an example of two superposed image modalities, one image modality representing a CT scan and the other image modality representing an ultrasound scan, according to an embodiment of the present disclosure; and

FIG. 6B depicts an example of the two superposed image modalities being optimally overlapped, according to an embodiment of the present disclosure.

## DETAILED DESCRIPTION

The present disclosure describes medical-device systems and methods that allow operators to perform navigation-assisted interventions using certain types of intervention instruments, such as needle-like instruments, and corresponding methods of optimizing registration between image modalities using visual or automatic termination criteria. Example applications may include needle biopsies, tumor ablations, catheter insertion, orthopedic interventions, and other instruments, all of which may use several types of image data. Typically, these instruments are inserted into a patient's body at very specific locations, orientations, and depths to reach predetermined target areas, where they perform an instrument-specific action or function, which may include tissue sampling, heating, cooling, liquid deposition, suction, or serving as a channel for other objects.

Clear Guide Medical has previously developed a novel visual tracking technology platform based on real-time camera-based computer vision, and embodied the tracking platform in products for ultrasound-based systems and computerized tomography (CT)-based systems for image/instrument guidance and multi-modality fusion. Certain aspects of this technology has already been described in U.S. Patent Application No. 13/648,245, U.S. Patent Application No. 14/092,755, U.S. Patent Application Serial Number 14/092,843, U.S. Patent Application Serial Number 14/508,223, U.S. Patent Application Serial Number 14/524,468, U.S. Patent Application Serial Number 14/524,570, and U.S. Patent Application Serial Number 14/689,849, which are hereby referenced.

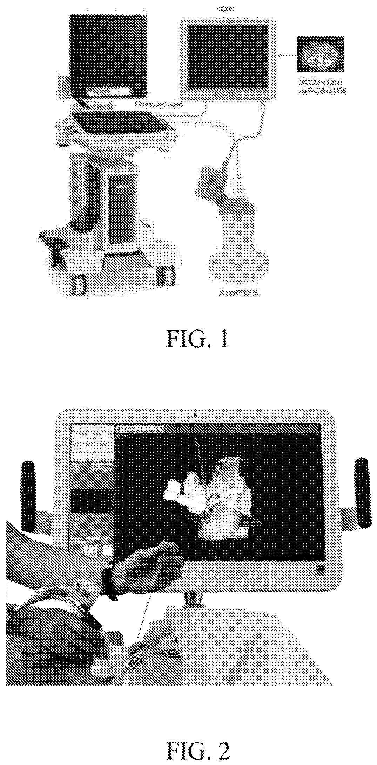

FIGs. 1 and 2 depict examples of the above-noted systems. FIG 1. depicts an example of an ultrasound system (Clear Guide SCENERGY system) having an ultrasound probe having mounted thereon a tracking device (e.g., one or more cameras) for tracking a position of the ultrasound probe on a patient's body. One or more cameras image objects in their field of view, detect and localize reference features for registration and tracking, reconstruct their poses in camera space, and register them to corresponding objects' poses in other image data sets. As it will described further in detail in the following paragraphs, the ultrasound system can be configured in accordance with embodiments of the present disclosure for navigating interventional instrumentation, such as surgical needles, and refining registration between image modalities.

FIG. 2 depicts a health practitioner inserting a needle into a patient's body while using an ultrasound system (Clear Guide Medical "SCENERGY" system) having the one or more cameras to track the position of the needle relative to patient's body, the ultrasound system providing an image displaying information about a path of the needle insertion within the patient's body. The Clear Guide "SCENERGY" system allows fusion of registration images of ultrasound and CT devices based on skin-attached "VisiMARKER" fiducials. These multi-modality markers comprise a radiopaque layer that is automatically segmented from radiographic imaging based on its known high intensity, and a visible checkerboard-like surface pattern that allows observing cameras to localize the marker in six degrees of freedom in camera coordinates (see also Olson E., AprilTag: A robust and flexible visual fiducial system, IEEE-ICRA 2011, the entire content of which is incorporated herein by reference).

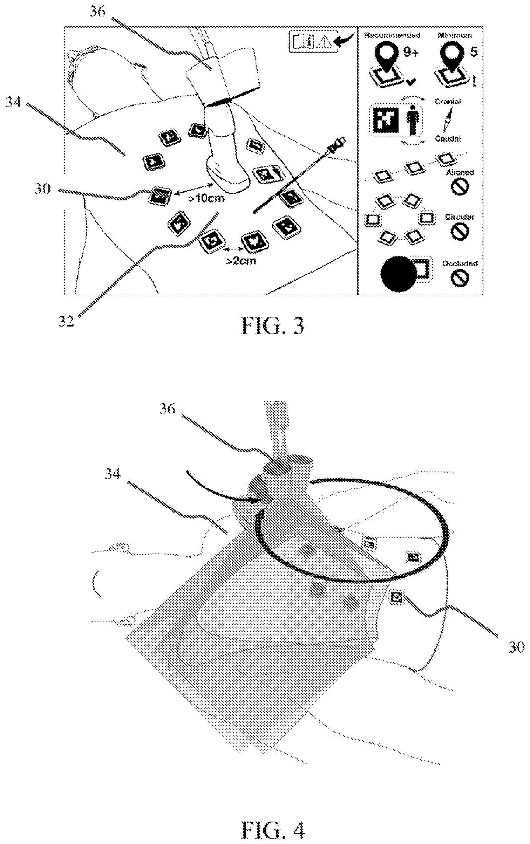

FIG. 3 shows a plurality of VisiMARKERs 30 that are applied in an irregular fashion around the intervention area 32 in a body 34 of a patient. The VisiMARKERS 30 are imaged together with the body 34 of the patient. The operator or health pratitioner moves the handheld camera head 36 in a sweeping motion above the patient surface, thus collecting marker observations.

FIG. 4 depicts a camera sweep wherein the camera captures a plurality of images including the VisiMARKERS 30. Consecutive observations of marker $n$-tuples (for $n \geq 2$) allow the successive insertion of those markers and their relative poses into a three-dimensional (3D) mesh. This observation mesh is then automatically registered onto the marker locations auto-segmented from CT by comparing all possible alignments of both point sets and choosing the most plausible transformation $M$ (see, U.S. Patent Application Serial Number 14/689,849 to Stolka et al). This allows the system to localize the camera 36 relative to the patient 34 (via direct observations of the markers 30), the camera 36 relative to the patient image data (via registration to the marker mesh), ultrasound relative to the camera 36 (via predetermined intrinsic device calibration), and therefore ultrasound to patient data.

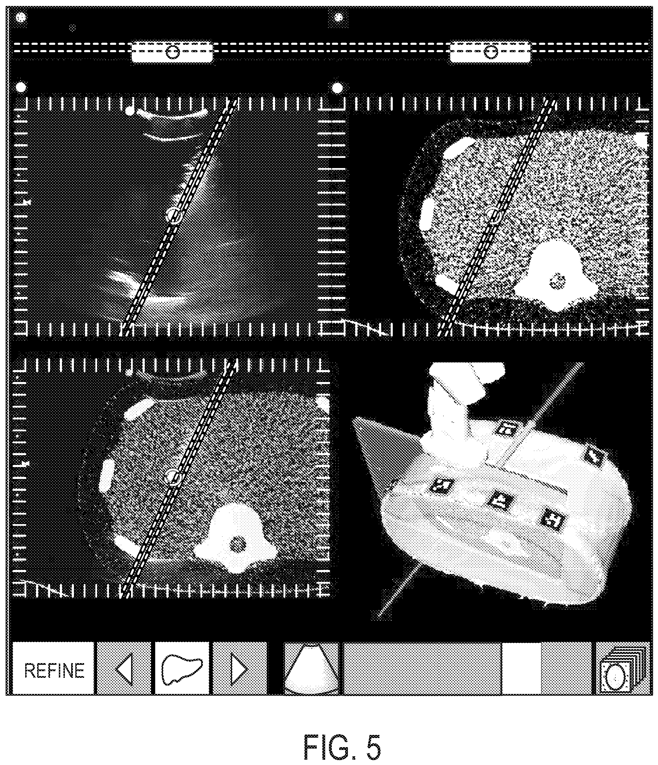

The system can then continuously extract slices from the patient data that spatially correspond to the current ultrasound slice, and show both overlaid on screen in a fusion view, as shown in FIG. 5. FIG. 5, at the lower right corner, illustrates a position of the markers 30 on the body 34 of the patient. When the handheld probe with the camera 36 moves across the intervention area 32, all imaging modalities show corresponding real-time fused views. The upper right and lower left corner of FIG. 5 are shown images corresponding to one image modality (e.g., CT scan image modality) superposed thereon a track of a surgical device (e.g., a needle) being inserted into the body of the patient. In the upper left corner of FIG. 5 is shown another image modality (e.g., an ultrasound scan image modality) superposed thereon a track of a surgical device (e.g., a needle) being inserted into the body of the patient.

**Manual Registration Refinement:** However, the above process for performing a "Visual Sweep" registration based on the position of the markers 30 is subject to noise including patient breathing artifacts during initial imaging or the Visual Sweep, marker drift or skin shift between imaging and registration, imperfect marker observations, internal tissue shift etc. These noise sources may deteriorate the registration quality, leading to noticeable displacement between registered image data and actual relevant anatomical features as viewed by real-time imaging such as ultrasound or fluoroscopy.

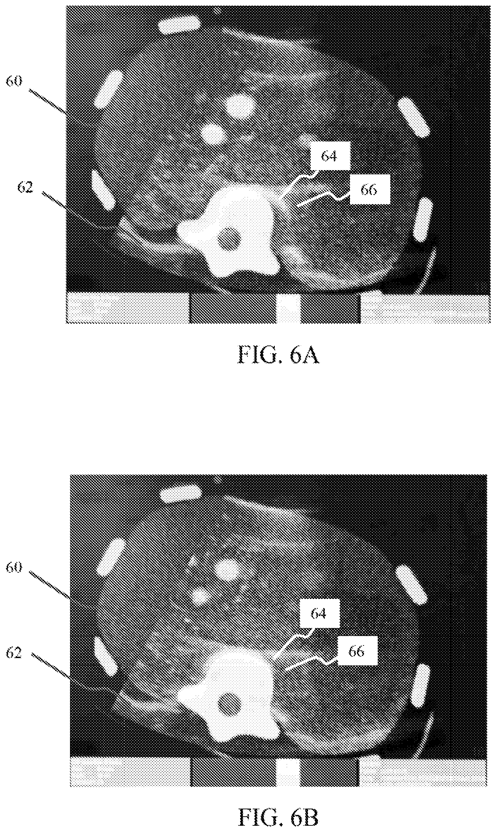

FIG. 6A depicts an example of two superposed image modalities, one image modality 60 representing a CT scan and the other image modality 62 representing an ultrasound scan, according to an embodiment of the present disclosure. As shown in FIG. 6A, the features shown in CT image 60 do not line up with corresponding features shown in ultrasound image 62. For example, a profile 64 of a backbone (simulating the backbone of a patient) shown in CT image 60 does not coincide with a profile 66 of the same backbone shown in ultrasound image 62. The two profiles 64 and 66 appear to be shifted relative to each other.

The term "real-time" imaging is used herein to mean (a) that the images are captured while the patient is imaged with the imaging system(s), (b) the time the patient is willing to wait on the imaging table, (c) the images are viewed within approximately one minute (e.g., less than 30 seconds) from capturing the image by a probe (e.g. ultrasound probe), or (d) the images are viewed within less than one second from capturing the images (i.e., relatively instantaneously).

Therefore, it may be desirable to adjust the initial, automatically determined registration matrix M to achieve better feature alignment. In an embodiment, one possible procedure to correct for misalignment is to allow an operator (e.g., medical practitioner) to perform a "manual registration refinement", as follows:

1. The operator observes relevant feature misalignment between two fused or superposed image modalities. For example, the operator may observe misalignment of profile 64 and profile 66 of the backbone, as shown in FIG. 6A.

2. The operator "unlocks" the real-time image modality while maintaining the static image modality "frozen" in a same position. For example, the operator may unlock the ultrasound image modality 62 while the CT image modality 60 remains static in position. For example, this can be performed by activating a button, switch or other feature on the ultrasound scanner system to allow the captured ultrasound image 62 to refresh or update in real-time and move relative to the captured CT scan image modality 60 which remains fixed in position.

3. The operator systematically moves the real-time image (e.g., by moving the ultrasound probe) such that relevant features optimally overlap features in the frozen image modality. For example, the operator may move the ultrasound probe until the profile 66 shown in the captured ultrasound image modality 62 coincides or overlaps with the profile 64 shown in the captured CT scan image modality 60. FIG. 6B depicts an example of the two superposed image modalities 60 and 62 being optimally overlapped, according to an embodiment of the present disclosure. The term "optimally overlapped" is used herein to mean that the image modalities 60 and 62 appear to coincide to the best visual assessment ability of the operator.

4. The operator then "re-locks" the real-time modality (e.g., the ultrasound image modality 62) to the static image modality (e.g., the CT scan image modality 60). For example, in an embodiment, the image modalities 60 and 62 can be both allowed to resume moving together in unison while remaining locked in position relative to each other.

By using the above procedure, the operator can guide image registration optimization both by manually searching for the optimal alignment pose, and by visually assessing alignment quality.

**Automatic Termination:** In another embodiment, the above-described manual refinement procedure may suffer from subjectivity as it depends on a visual alignment assessment of the operator. Furthermore, the above-described manual alignment may also have usability shortcomings in that multiple interaction types during dexterous high-accuracy actions may be needed. As a result, in order to remedy the above deficiencies and further improve upon the above described manual/visual refinement procedure, a further alignment procedure is employed herein that removes the subjective visual assessment of the operator. In an embodiment, the alignment procedure includes:

1. The operator observes relevant feature misalignment between two fused or superposed image modalities.

2. The operator "unlocks" the real-time image modality (e.g., unlocks the ultrasound image modality 62) while the static image modality (e.g., the CT scan image modality 60) remains "frozen" in place.

3. The operator systematically moves the real-time image (e.g., moves the ultrasound image modality 62 by moving the ultrasound probe) such that relevant features optimally overlap corresponding ones in the frozen modality (e.g., the CT scan image modality 60).

4. The system automatically computes an image registration quality (IRQ) metric in real-time and displays the computed IRQ metric value to the operator as feedback. The operator may use the IRQ metric as guidance in the search for an optimum alignment. In an embodiment, the greater the value of IRQ achieved, the better is the alignment achieved between the two images modalities (e.g., CT image modality 60 and ultrasound image modality 62). The system continuously stores the maximum-to-date achieved IRQ value, and the corresponding alignment pose that allowed for this maximum IRQ value.

5. The system compares a first IRQ value obtained in a first attempted alignment and a second IRQ value obtained with a second subsequently attempted alignment and selects an alignment among the first attempted alignment and the second attempted alignment having the greater IRQ value among the first and second IRQ values as the best alignment to date.

6. The system repeats this procedure and select the best alignment that achieves a greater IRQ value.

7. If the system determines that, after a certain number of iterations, no improvement is possible any more, or no improvement expected any more based on the IRQ metric's trend (e.g., the IRQ value plateaued or constantly falling in subsequent attempts for alignment), or after the IRQ value has reached a certain threshold value (e.g., selected by the user), alignment refinement is automatically terminated. A previously stored pose having the maximum-to-date achieved IRQ value (or the initial registration if no improvement was found) is selected as being the optimum alignment.

8. The system automatically "re-locks" the real-time modality (e.g., the ultrasound image modality 62) to the static image modality (e.g., the CT scan image modality 60). For example, in an embodiment, the image modalities 60 and 62 can be both allowed to resume moving together in unison while remaining locked in position relative to each other. The term "automatically" is used herein to indicate that the system performs a certain function without manual interference from the user or operator.

9. Alternatively, the system provides an audio or visual feedback to the operator to inform the operator that the optimum alignment is reached. The operator then can manually "re-lock" the real-time modality (e.g., the ultrasound image modality 62) to the static image modality (e.g., the CT scan image modality 60). For example, in an embodiment, the image modalities 60 and 62 can be both allowed to resume moving together in unison while remaining locked in position relative to each other.

In the above paragraphs, it is described that the maximum value of the IRQ determines the best or optimum alignment between the images modalities. However, as it can be appreciated, in another embodiment, another type of IRQ metric can also be selected or generated such that the smaller the value of IRQ achieved, the better is the alignment achieved between the two images modalities (e.g., CT image modality 60 and ultrasound image modality 62). In this case, instead of tracking or following the maximum value of the IRQ metric, the minimum value of the IRQ metric provides the indication when the optimum alignment between the image modalities is achieved.

This approach has the benefit of relieving the operator of the alignment assessment burden. The system computes the image registration quality (IRQ) metric by correlating the respective current modality images (real-time and "frozen" static slices). Depending on combination of image modalities, the IRQ function may be straightforward image correlation for identical modalities, but may require preprocessing in other cases. For example, ultrasound is a boundary-based modality, whereas CT is a volume-based modality. Initially, the CT scanner's vertical gradient image may be computed (to approximate the ultrasound's top-down insonification and the resultant highlighting of horizontal tissue interfaces with large acoustic impedance jumps). Then, a correlation or better mutual-information-based (see, also Wells et al., "Multi-Modal Volume Registration by Maximization of Mutual Information," Medical Image Analysis, 1996, the entire content of which is incorporated herein by reference) may be used to estimate alignment quality.

Since an initial visual sweep registration is almost guaranteed to result in a registration very close to the global optimum of the IRQ function, one may assume that the manual sampling of the pose space by the operator consistently results in higher values closer to the global optimum, and in lower values further away. This safely allows the system to automatically terminate the optimization and return the optimum-to-date pose as the result.

This approach may be also applied in cases where the real-time imaging modality is substituted or combined by one or more other image modalities or data sets (such as other CT, CBCT, MRI, etc. volumes). The manual refinement may be performed with real-time imaging against any other operator or automatically selected static volumes (e.g., by automatically choosing the lowest-quality matched data set for unlocking), as well as with static volumes against other static volumes, using similar interaction and computation approaches as outlined above.

As it can be appreciated from the above paragraphs, in an embodiment of the present disclosure, there is provided a system for surgical image guidance. The system includes a first imaging device and an image processing system operatively connected to the first imaging device. The image processing system is configured to: 1) receive real-time image data of a patient from the first imaging device; 2) receive secondary image data from a second imaging device; 3) produce composite image data based on the real-time image data and the secondary image data; and 4) produce enhanced composite image data by improving an alignment of physical structures in a real-time image based on the real-time image data with corresponding physical structures in a secondary image based on the secondary image data. The image processing system is further configured to operate in an unlocked mode in which the real-time image is free to move relative to the secondary image and a locked mode wherein the real-time image and the secondary image are locked relative to each other to prevent relative movement therebetween. The imaging device is configured to be able to provide information to the image processing system to cause the image processing system to operate in the unlocked mode to enable movement of the real-time image relative to the secondary image.

In an embodiment, the system may further include a display device configured to receive and display the composite image data. In an embodiment, the first imaging device can be an ultrasound device and the second imaging device can be a CT scan device or an MRI device or a three-dimensional medical imaging device.

In an embodiment, the image processing system can be configured to allow correcting of a misalignment between the real-time image data relative to the secondary image data until an operator determines that the real-time image data coincides with the secondary image data.

In an embodiment, the image processing system may include an input device configured to receive an input from an operator to put the image processing system in the unlocked mode to allow the real-time image to update and move in position relative to the secondary image. In an embodiment, the input device can be further configured to receive an input from the operator to put the image processing system in the locked mode to prevent relative movement between the real-time image and the secondary image.

In an embodiment, the image processing system can be configured to compute an image registration quality (IRQ) metric continuously. The IRQ metric quantifies a degree of the alignment of the physical structures in the real-time image with the corresponding physical structures in the secondary image. In an embodiment, the image processing system can be configured to determine whether the IRQ metric meets a predetermined threshold value or a dynamically determined threshold value, and provide, based on the determination, a feedback signal to an operator or to automatically put the image processing system in the locked mode to prevent relative movement between the real-time image and the secondary image. In an embodiment, the image processing system can be configured to display the IRQ metric as feedback. In an embodiment, the dynamically determined threshold value is a maximum or a minimum IRQ value. In an embodiment, the image processing system can be configured to store the IRQ metric and a corresponding alignment pose that achieved the IRQ metric. In an embodiment, the image processing system can be configured to compare a first IRQ metric obtained in a first attempted alignment pose with a second IRQ metric obtained with a second attempted alignment pose, and to store as an alignment pose the first attempted alignment pose or the second attempted alignment pose that achieved a best IRQ value among the first IRQ metric and the second IRQ metric. For example, the best IRQ metric corresponds to a higher IRQ metric in the first and second IRQ metrics, a lower IRQ metric in the first and second IRQ metrics, or an IRQ metric among the first and second IRQ metrics that is closest to the predetermined threshold value.

In an embodiment, the image processing system can be configured to automatically lock an alignment between the physical structures in the real-time image with the corresponding physical structures in the secondary image, if, after a certain number of comparison iterations, no improvement in alignment is possible any more, or no improvement in alignment is expected any more based on a trend of the IRQ metric, or after the IRQ metric has reached the predetermined threshold value, and select a previously stored alignment pose having a best achieved IRQ metric as an optimum alignment pose. For example, the IRQ metric can be determined by performing a two-dimensional correlation between the real-time image data and the corresponding secondary image data. The two-dimensional cross-correlation between the real-time image data and the corresponding secondary image data can be performed according to the following equation:

$$r_{ij} = \frac{\sum_m \sum_n [f(m+i, n+j) - \bar{f}][g(m,n) - \bar{g}]}{\sqrt{\sum_m \sum_n [f(m,n) - \bar{f}]^2 \sum_m \sum_n [g(m,n) - \bar{g}]^2}}$$

where $f(m,n)$ and $g(m,n)$ are respective image pixel values in the real-time image data and corresponding secondary image data at locations $m,n$, with $i=j=0$ for determining said correlation at the current alignment pose.

As it can be appreciated from the above paragraphs, in an embodiment of the present disclosure, there is also provided a method for surgical image guidance. The method includes 1) receiving, by an image processing system, real-time image data of a patient from the first imaging device; 2) receiving, by the image processing system, secondary image data from a second imaging device; 3) producing, by the image processing system, composite image data based on the real-time image data and the secondary image data; and 4) producing, by the image processing system, enhanced composite image data by improving an alignment of physical structures in a real-time image based on the real-time image data with corresponding physical structures in a secondary image based on the secondary image data. The method also includes receiving, by the image processing system, a command permit moving the real-time image relative to the secondary image when the image processing system is operating in an unlocked mode in which the real-time image is free to move relative to the secondary image. The method further includes receiving, by the processing system, a command to put the processing system in a locked operating mode so as to prevent relative movement between the real-time image and the secondary image.

In an embodiment, the method further includes allowing for correction, by the processing system, for a misalignment between the real-time image data relative to the secondary image data until an operator determines that the real-time image data coincides with the secondary image data. In an embodiment, the method includes computing, by the processing system, an image registration quality (IRQ) metric continously, the IRQ metric quantifying a degree of the alignment of the physical structures in the real-time image with the corresponding physical structures in the secondary image. In an embodiment, the method further includes determining whether the IRQ metric meets a predetermined threshold value or a dynamically determined threshold value; and providing, based on the determination, a feedback signal to an operator or to automatically put the image processing system in the locked mode to prevent relative movement between the real-time image and the secondary image. The method may also include providing the IRQ metric as feedback to the operator. In an embodiment, the dynamically determined threshold value can be a maximum or a minimum IRQ value. The method also includes storing the IRQ metric and a corresponding alignment pose that achieved the IRQ metric.

In an embodiment, the method may also include comparing a first IRQ metric obtained in a first attempted alignment pose with a second IRQ metric obtained in a second attempted alignment pose, and storing as an alignment pose the first attempted alignment pose or the second attempted alignment pose that achieved a best IRQ value among the first IRQ metric and the second IRQ metric. For example, best IRQ metric can correspond to a higher IRQ metric in the first and second IRQ metrics, a lower IRQ metric in the first and second IRQ metrics, or an IRQ metric among the first and second IRQ metrics that is closest to the predetermined threshold value.

In an embodiment, the method may also include automatically locking an alignment between the physical structures in the real-time image with the corresponding physical structures in the secondary image, if, after a certain number of comparison iterations, no improvement in alignment is possible any more, or no improvement in alignment is expected any more based on a trend of the IRQ metric, or after the IRQ metric has reached the predetermined threshold value, and selecting a previously stored alignment pose having a best achieved IRQ metric as an optimum alignment pose.

According to an embodiment of the present disclosure, there is also provided a non-transitory processor-readable medium having instructions stored thereon, which when executed by one or more processors, cause the one or more processors to implement the above method.

The foregoing detailed description of embodiments includes references to the drawings or figures, which show illustrations in accordance with example embodiments. The embodiments described herein can be combined, other embodiments can be utilized, or structural, logical and operational changes can be made without departing from the scope of what is claimed. The foregoing detailed description is, therefore, not to be taken in a limiting sense, and the scope is defined by the appended claims and their equivalents. It should be evident that various modifications and changes can be made to these embodiments without departing from the broader spirit and scope of the present disclosure. Accordingly, the specification and drawings are to be regarded in an illustrative rather than a restrictive sense.

Present teachings may be implemented using a variety of technologies. For example, certain aspects of this disclosure may be implemented using electronic hardware, computer software, or any combination thereof. Whether such elements are implemented as hardware or software depends upon the particular application and design constraints imposed on the overall system. By way of example, the electronic hardware, or any portion of electronic hardware may be implemented with a processing system that includes one or more processors. Examples of processors include microprocessors, microcontrollers, Central Processing Units (CPUs), digital signal processors (DSPs), field programmable gate arrays (FPGAs), programmable logic devices (PLDs), state machines, gated logic, discrete hardware circuits, and other suitable hardware configured to perform various functions described throughout this disclosure. One or more processors in the processing system may execute software, firmware, or middleware (collectively referred to as "software"). The term "software" shall be construed broadly to mean instructions, instruction sets, code, code segments, program code, programs, subprograms, software components, applications, software applications, software packages, routines, subroutines, objects, executables, threads of execution, procedures, functions, etc., whether referred to as software, firmware, middleware, microcode, hardware description language, or otherwise. In certain embodiments, the electronic hardware can also include designed application-specific integrated circuits (ASICs), programmable logic devices, or various combinations thereof. The processing system can refer to a computer (e.g., a desktop computer, tablet computer, laptop computer), cellular phone, smart phone, and so forth. The processing system can also include one or more input devices, one or more output devices (e.g., a display), memory, network interface, and so forth.

If certain functions described herein are implemented in software, the functions may be stored on or encoded as one or more instructions or code on a non-transitory computer-readable medium. Computer-readable media includes computer storage media. Storage media may be any available media that can be accessed by a computer. By way of example, and not limitation, such computer-readable media can comprise a random-access memory (RAM), a read-only memory (ROM), an electrically erasable programmable ROM (EEPROM), compact disk ROM (CD-ROM) or other optical disk storage, magnetic disk storage, solid state memory, or any other data storage devices, combinations of the aforementioned types of computer-readable media, or any other medium that can be used to store computer executable code in the form of instructions or data structures that can be accessed by a computer.

For purposes of this patent document, the terms "or" and "and" shall mean "and/or" unless stated otherwise or clearly intended otherwise by the context of their use. The term "a" shall mean "one or more" unless stated otherwise or where the use of "one or more" is clearly inappropriate. The terms "comprise," "comprising," "include," and "including" are interchangeable and not intended to be limiting. For example, the term "including" shall be interpreted to mean "including, but not limited to."

Although embodiments have been described with reference to specific example embodiments, it will be evident that various modifications and changes can be made to these example embodiments without departing from the broader spirit and scope of the present application. Accordingly, the specification and drawings are to be regarded in an illustrative rather than a restrictive sense.

The embodiments illustrated and discussed in this specification are intended only to teach those skilled in the art how to make and use the invention. In describing embodiments of the disclosure, specific terminology is employed for the sake of clarity. However, the disclosure is not intended to be limited to the specific terminology so selected. The above-described embodiments of the disclosure may be modified or varied, without departing from the invention, as appreciated by those skilled in the art in light of the above teachings, as long as the result still falls within the scope of the appended claims. It is therefore to be understood that, within the scope of the claims and their equivalents, the invention may be practiced otherwise than as specifically described. For example, it is to be understood that the present disclosure contemplates that, to the extent possible, one or more features of any embodiment can be combined with one or more features of any other embodiment.

**Claims**

1. A system for surgical image guidance, comprising:

a tracking camera (36);
an ultrasound imaging device capable of real-time imaging of a patient's body (34), said ultrasound imaging device mounted to the tracking camera;
a tracking system configured to receive optical image data from the tracking camera and to determine a position of the ultrasound imaging device relative to the patient's body, based on a plurality of fiducial markers (30) attached to the patient's body and visible in the optical image data;
an image processing system operatively connected to the ultrasound imaging device and the tracking system, the image processing system configured to:

receive a real-time ultrasound image (62) of the patient from the ultrasound imaging device and a three-dimensional, 3D, image dataset (60) of the patient from another imaging device, said plurality of fiducial markers also visible in the 3D image dataset;
determine a position of each of the plurality of fiducial markers relative to the 3D image dataset;
perform a registration of the real-time ultrasound image with the 3D image dataset, based on the position of the ultrasound imaging device relative to the patient's body and on the position of the fiducial markers relative to the 3D image dataset;
based on the registration of the real-time ultrasound image with the 3D image dataset, extract a spatially corresponding image from the 3D image dataset;
produce a composite image based on an overlay of the real-time image with the extracted image from the 3D image dataset;
provide a mode in which the real-time ultrasound image is free to move relative to the extracted image from the 3D image dataset;
refine the registration by receiving at least one additional real-time ultrasound image wherein physical structures (64, 66) in the extracted image overlap with corresponding physical structures (64, 66) in the at least one additional real-time ultrasound image; and
provide automatic or manual means to enter a mode in which the real-time ultrasound image and the extracted image from the 3D image dataset are locked relative to each other to prevent relative movement therebetween; and

a display device configured to receive the composite image from the image processing system and display the composite image.

2. The system according to claim 1, wherein the registration can be refined in a manual mode in which an operator of the ultrasound imaging device visually determines that the physical structures in the extracted image overlap with the corresponding physical structures in the additional real-time image, using the composite image displayed on the display device.

3. The system according to claim 1, wherein the registration can be refined in an automatic mode in which the image processing system is further configured to:

compute an image registration quality, IRQ, metric that quantifies a quality of an alignment of the physical structures in the extracted image with the corresponding physical structures in the additional real-time image; and
provide on the display device the IRQ metric as real-time feedback to an operator of the ultrasound imaging device,
wherein determining that the physical structures (64, 66) in the extracted image overlap with corresponding physical structures in the additional real-time image is based on an optimized value of the computed IRQ metric.

4. The system according to claim 3, wherein the image processing system is further configured to:

determine whether the IRQ metric meets a predetermined threshold value or a dynamically determined threshold value; and
based on the determination, automatically configure the image processing system to require the at least one additional real-time image to spatially correspond to the extracted image.

5. The system according to claim 4, wherein

the dynamically determined threshold value is one of a maximum IRQ value and a minimum IRQ value, or
the image processing system is configured to store the IRQ metric and a corresponding alignment pose that achieved the IRQ metric, or
the image processing system is configured to compare a first IRQ metric obtained in a first attempted alignment pose with a second IRQ metric obtained in a second attempted alignment pose and to store as an alignment pose the first alignment pose or the second attempted alignment pose that achieved a best IRQ value among the first IRQ metric and the second IRQ metric.

6. The system according to claim 5, wherein the best IRQ metric corresponds to one of a higher IRQ metric in the first and second IRQ metrics, a lower IRQ metric in the first and second IRQ metrics, and an IRQ metric among the first and second IRQ metrics that is closest to the predetermined threshold value, and
optionally, the image processing system is configured to:

automatically lock an alignment between the physical structures in the at least one additional real-time image with the corresponding physical structures in the extracted image when after a certain number of comparison iterations no further improvement in alignment is possible, or no further improvement in alignment is expected based on a trend of the IRQ metric, or after the IRQ metric has reached the predetermined threshold value, and select a previously stored alignment pose having a best achieved IRQ metric as an optimum alignment pose.

7. The system according to claim 1, wherein the image processing system comprises an input device configured to receive a first input from an operator to configure the image processing system to allow the at least one additional real-time image to update without spatially corresponding to the extracted image, and further receive a second input from the operator to configure the image processing system to require the at least one additional real-time image to spatially correspond to the extracted image.

8. The system according to claim 3, wherein the IRQ metric is computed by performing a two-dimensional cross-correlation between the additional real-time image and the extracted image.

9. The system according to claim 8, wherein the two-dimensional cross-correlation between the additional real-time image and the extracted image is performed according to the following equation:

$$r_{ij} = \frac{\sum_m \sum_n [f(m+i, n+j) - \bar{f}][g(m,n) - \bar{g}]}{\sqrt{\sum_m \sum_n [f(m,n) - \bar{f}]^2 \sum_m \sum_n [g(m,n) - \bar{g}]^2}}$$

where *f(m,n)* and *g(m,n)* are respective image pixel values in the additional real-time image and the extracted image at locations *m,n,* with *i=j=0* for determining said correlation.

10. The system according to claim 1, wherein the other imaging device is one of a computed tomography, CT, scan device, a magnetic resonance imaging, MRI, device, and a three-dimensional medical imaging device.

11. A method for surgical image guidance comprising:

receiving by an image processing system a real-time ultrasound image (62) of a patient from an ultrasound imaging device, a three-dimensional (3D) image dataset (60) of the patient from another imaging device, and a position of the ultrasound imaging device relative to the patient's body from a tracking system receiving optical image data from a tracking camera, wherein a plurality of fiducial markers are visible in the 3D image dataset and visible in the optical image data;
determining a position of each of the plurality of fiducial markers relative to the 3D image dataset;
performing a registration of the real-time ultrasound image with the 3D image dataset, based on the position of the ultrasound imaging device relative to the patient's body and on the position of the fiducial markers relative to the 3D image dataset;
based on the registration of the real-time ultrasound image with the 3D image dataset, extracting a spatially corresponding image from the 3D image dataset;
producing by the image processing system a composite image based on an overlay of the real-time image with the extracted image from the 3D image dataset;

providing a mode in which the real-time ultrasound image is free to move relative to the extracted image from the 3D image dataset;

refining the registration by receiving at least one additional real-time ultrasound image wherein physical structures (64, 66) in the extracted image overlap with corresponding physical structures (64, 66) in the additional real-time ultrasound image;

providing automatic or manual means to enter a mode in which the real-time ultrasound image and the extracted image from the 3D image dataset are locked relative to each other to prevent relative movement therebetween; and

displaying the composite image on a display device.

12. The method according to claim 11, wherein the registration is refined in a manual mode in which an operator of the ultrasound imaging device visually determines that the physical structures in the extracted image overlap with the corresponding physical structures in the additional real-time image using the composite image displayed on the display device.

13. The method according to claim 11, wherein the registration is refined in an automatic mode in which the method further comprises computing an image registration quality, IRQ, metric, that quantifies a quality of an alignment of the physical structures in the extracted image with the corresponding physical structures in the additional real-time image; and

providing on the display device the IRQ metric as real-time feedback to an operator of the ultrasound imaging device,

wherein determining that the physical structures (64, 66) in the extracted image overlap with corresponding physical structures in the additional real-time image is based on an optimized value of the computed IRQ metric.

14. The method according to claim 13, further comprising:

determining whether the IRQ metric meets a predetermined threshold value or a dynamically determined threshold value; and

based on the determination, requiring the at least one additional real-time image to spatially correspond to the extracted image.

15. The method according to claim 14, wherein the dynamically determined threshold value is one of a maximum IRQ value and a minimum IRQ value, and

optionally, the method further comprises storing the IRQ metric and a corresponding alignment pose that achieved the IRQ metric, and

optionally, the method further comprises comparing a first IRQ metric obtained in a first attempted alignment pose with a second IRQ metric obtained in a second attempted alignment pose, and storing as an alignment pose the first attempted alignment pose or the second attempted alignment pose that achieved a best IRQ value among the first IRQ metric and the second IRQ metric; or

wherein the best IRQ metric corresponds to one of a higher IRQ metric in the first and second IRQ metrics, a lower IRQ metric in the first and second IRQ metrics, and an IRQ metric among the first and second IRQ metrics that is closest to the predetermined threshold value, and

optionally, the method further comprises automatically locking an alignment between the physical structures in the at least one additional real-time image with the corresponding physical structures in the extracted image when after a certain number of comparison iterations no further improvement in alignment is possible, or no further improvement in alignment is expected based on a trend of the IRQ metric, or after the IRQ metric has reached the predetermined threshold value, and

selecting a previously stored alignment pose having a best achieved IRQ metric as an optimum alignment pose.

**Patentansprüche**

1. System zur chirurgischen Bildführung, umfassend:

eine Tracking-Kamera (36);
eine Ultraschall-Bildgebungsvorrichtung, die geeignet ist, den Körper eines Patienten (34) in Echtzeit abzubilden, wobei die Ultraschall-Bildgebungsvorrichtung an der Tracking-Kamera angebracht ist;

ein Trackingsystem, das derart konfiguriert ist, dass es optische Bilddaten von der Tracking-Kamera empfängt und eine Position der Ultraschall-Bildgebungsvorrichtung relativ zum Körper des Patienten basierend auf einer Vielzahl von Referenzmarkierungen (30) bestimmt, die am Körper des Patienten angebracht sind und in den optischen Bilddaten sichtbar sind;

ein Bildverarbeitungssystem, das mit der Ultraschall-Bildgebungsvorrichtung und dem Tracking-System wirkverbunden ist, wobei das Bildverarbeitungssystem eingerichtet ist:

zum Empfangen eines Echtzeit-Ultraschallbildes (62) des Patienten von der Ultraschall-Bildgebungsvorrichtung und eines dreidimensionalen (3D) Bilddatensatzes (60) des Patienten von einer anderen Bildgebungsvorrichtung, wobei die Vielzahl von Referenzmarkierungen auch in dem 3D-Bilddatensatz sichtbar ist;

zum Bestimmen einer Position jeder der Vielzahl von Referenzmarkierungen relativ zu dem 3D-Bilddatensatz;

zum Durchführen einer Registrierung des Echtzeit-Ultraschallbildes mit dem 3D-Bilddatensatz, basierend auf der Position der Ultraschall-Bildgebungsvorrichtung relativ zum Körper des Patienten und auf der Position der Referenzmarkierungen relativ zum 3D-Bilddatensatz;

zum Extrahieren eines räumlich entsprechenden Bildes aus dem 3D-Bilddatensatz, basierend auf der Registrierung des Echtzeit-Ultraschallbildes mit dem 3D-Bilddatensatz;

zum Erstellen eines zusammengesetzten Bildes basierend auf einer Überlagerung des Echtzeitbildes mit dem aus dem 3D-Bilddatensatz extrahierten Bild;

zum Bereitstellen eines Modus, in dem sich das Echtzeit-Ultraschallbild relativ zu dem aus dem 3D-Bilddatensatz extrahierten Bild frei bewegen kann;

zum Verfeinern der Registrierung durch Empfangen von mindestens einem zusätzlichen Echtzeit-Ultraschallbild, wobei sich physische Strukturen (64, 66) in dem extrahierten Bild mit entsprechenden physischen Strukturen (64, 66) in dem mindestens einen zusätzlichen Echtzeit-Ultraschallbild überlappen; und

zum Bereitstellen von automatischen oder manuellen Mitteln, um einen Modus aufzurufen, in dem das Echtzeit-Ultraschallbild und das aus dem 3D-Bilddatensatz extrahierte Bild relativ zueinander fixiert sind, um eine Relativbewegung zwischen diesen Bildern zu verhindern; und umfassend eine Anzeigevorrichtung, die derart konfiguriert ist, dass sie das zusammengesetzte Bild von dem Bildverarbeitungssystem empfängt und anzeigt.

2. System nach Anspruch 1, wobei die Registrierung in einem manuellen Modus verfeinert werden kann, in dem eine Bedienungsperson der Ultraschall-Bildgebungsvorrichtung visuell feststellt, dass sich die physischen Strukturen in dem extrahierten Bild mit den entsprechenden physischen Strukturen in dem zusätzlichen Echtzeitbild überlappen, wobei das zusammengesetzte Bild verwendet wird, das auf der Anzeigevorrichtung angezeigt wird.

3. System nach Anspruch 1, wobei die Registrierung in einem automatischen Modus verfeinert werden kann, in dem das Bildverarbeitungssystem ferner eingerichtet ist:

zum Berechnen einer Bildregistrierungsqualitäts-Metrik (IRQ-Metrik), die eine Qualität einer Ausrichtung der physischen Strukturen in dem extrahierten Bild mit den entsprechenden physischen Strukturen in dem zusätzlichen Echtzeitbild quantifiziert; und

zum Bereitstellen der IRQ-Metrik als Echtzeit-Rückmeldung an eine Bedienungsperson des Ultraschall-Bildgebungsgeräts auf der Anzeigevorrichtung,

wobei die Feststellung, dass sich die physischen Strukturen (64, 66) in dem extrahierten Bild mit entsprechenden physischen Strukturen in dem zusätzlichen Echtzeitbild überlappen, auf einem optimierten Wert der berechneten IRQ-Metrik basiert.

4. System nach Anspruch 3, wobei das Bildverarbeitungssystem ferner eingerichtet ist:

zum Feststellen, ob die IRQ-Metrik einen vorbestimmten Schwellenwert oder einen dynamisch bestimmten Schwellenwert erfüllt; und,

basierend auf der Feststellung, zum automatischen Konfigurieren des Bildverarbeitungssystems derart, dass das mindestens eine zusätzliche Echtzeitbild dem extrahierten Bild räumlich entsprechen muss.

5. System nach Anspruch 4, wobei

der dynamisch bestimmte Schwellenwert einer von einem maximalen IRQ-Wert und einem minimalen IRQ-Wert ist oder

das Bildverarbeitungssystem dazu eingerichtet ist, die IRQ-Metrik und eine entsprechende Ausrichtungspose, die die IRQ-Metrik erzielt hat, zu speichern oder

das Bildverarbeitungssystem dazu eingerichtet ist, eine erste IRQ-Metrik, die in einer ersten versuchten Ausrichtungspose erhalten wurde, mit einer zweiten IRQ-Metrik, die in einer zweiten versuchten Ausrichtungspose erhalten wurde, zu vergleichen und als Ausrichtungspose die erste Ausrichtungspose oder die zweite versuchte Ausrichtungspose, die unter der ersten IRQ-Metrik und der zweiten IRQ-Metrik den besten IRQ-Wert erreicht hat, zu speichern.

6. System nach Anspruch 5, wobei die beste IRQ-Metrik einer der folgenden IRQ-Metriken entspricht: einer höheren IRQ-Metrik in der ersten und zweiten IRQ-Metrik, einer niedrigeren IRQ-Metrik in der ersten und zweiten IRQ-Metrik und zwischen der ersten und zweiten IRQ-Metrik einer IRQ-Metrik, die dem vorgegebenen Schwellenwert am nächsten ist, und wobei

das Bildverarbeitungssystem optional dazu eingerichtet ist:

eine Ausrichtung zwischen den physischen Strukturen in dem mindestens einen zusätzlichen Echtzeitbild mit den entsprechenden physischen Strukturen in dem extrahierten Bild zu fixieren, wenn nach einer bestimmten Anzahl von Vergleichswiederholungen keine weitere Verbesserung der Ausrichtung möglich ist oder basierend auf einer Tendenz der IRQ-Metrik keine weitere Verbesserung der Ausrichtung erwartet wird oder nachdem die IRQ-Metrik den vorgegebenen Schwellenwert erreicht hat, und

eine zuvor gespeicherte Ausrichtungspose mit einer erzielten besten IRQ-Metrik als optimale Ausrichtungspose auszuwählen.

7. System gemäß Anspruch 1, wobei das Bildverarbeitungssystem eine Eingabevorrichtung umfasst, die so konfiguriert ist, dass sie von einer Bedienungsperson eine erste Eingabe empfängt, um das Bildverarbeitungssystem derart einzurichten, dass es die Aktualisierung des mindestens einen zusätzlichen Echtzeitbildes ermöglicht, ohne räumlich dem extrahierten Bild zu entsprechen, und dass es von der Bedienungsperson ferner eine zweite Eingabe empfängt, um das Bildverarbeitungssystem derart einzurichten, dass das mindestens eine zusätzliche Echtzeitbild dem extrahierten Bild räumlich entsprechen muss.

8. System nach Anspruch 3, wobei die IRQ-Metrik durch Ausführen einer zweidimensionalen Kreuzkorrelation zwischen dem zusätzlichen Echtzeitbild und dem extrahierten Bild berechnet wird.

9. System nach Anspruch 8, wobei die zweidimensionale Kreuzkorrelation zwischen dem zusätzlichen Echtzeitbild und dem extrahierten Bild gemäß der folgenden Gleichung ausgeführt wird:

$$ r_{ij} = \frac{\sum_m \sum_n [f(m+i, n+j) - \bar{f}][g(m,n) - \bar{g}]}{\sqrt{\sum_m \sum_n [f(m,n) - \bar{f}]^2 \sum_m \sum_n [g(m,n) - \bar{g}]^2}} $$

wobei $f(m,n)$ und $g(m,n)$ jeweilige Bildpixelwerte in dem zusätzlichen Echtzeitbild und dem extrahierten Bild an den Stellen $m,n$ sind, mit $i=j=0$, um die Korrelation zu bestimmen.

10. System nach Anspruch 1, wobei die andere Bildgebungsvorrichtung eine Computertomographie(CT)-Scanvorrichtung, eine Magnetresonanzbildgebungs(MRI)-Vorrichtung und eine dreidimensionale medizinische Bildgebungsvorrichtung ist.

11. Verfahren zur chirurgischen Bildführung, umfassend:

Empfangen eines Echtzeit-Ultraschallbildes (62) eines Patienten von einer Ultraschall-Bildgebungsvorrichtung, eines dreidimensionalen (3D) Bilddatensatzes (60) des Patienten von einer anderen Bildgebungsvorrichtung und einer Position der Ultraschall-Bildgebungsvorrichtung relativ zum Körper des Patienten von einem Tracking-System, das optische Bilddaten von einer Tracking-Kamera empfängt, wobei eine Vielzahl von Referenzmarkierungen in dem 3D-Bilddatensatz und in den optischen Bilddaten sichtbar sind;

Bestimmen einer Position jeder der mehreren Referenzmarkierungen relativ zu dem 3D-Bilddatensatz;

Durchführen einer Registrierung des Echtzeit-Ultraschallbildes mit dem 3D-Bilddatensatz, basierend auf der

14

Position der Ultraschall-Bildgebungsvorrichtung relativ zu dem Körper des Patienten und auf der Position der Referenzmarkierungen relativ zu dem 3D-Bilddatensatz;

basierend auf der Registrierung des Echtzeit-Ultraschallbildes mit dem 3D-Bilddatensatz, Extrahieren eines räumlich entsprechenden Bildes aus dem 3D-Bilddatensatz;

Erstellen eines zusammengesetzten Bildes durch das Bildverarbeitungssystem, basierend auf einer Überlappung des Echtzeitbildes mit dem aus dem 3D-Bilddatensatz extrahierten Bild;

Bereitstellen eines Modus, in dem sich das Echtzeit-Ultraschallbild relativ zu dem aus dem 3D-Bilddatensatz extrahierten Bild frei bewegen kann;

Verfeinern der Registrierung durch Empfangen von mindestens einem zusätzlichen Echtzeit-Ultraschallbild, wobei sich physische Strukturen (64, 66) in dem extrahierten Bild mit entsprechenden physischen Strukturen (64, 66) in dem zusätzlichen Echtzeit-Ultraschallbild überlappen;

Bereitstellen automatischer oder manueller Mittel zum Aufrufen eines Modus, in dem das Echtzeit-Ultraschallbild und das aus dem 3D-Bilddatensatz extrahierte Bild relativ zueinander fixiert sind, um eine Relativbewegung zwischen den Bildern zu verhindern; und

Anzeigen des zusammengesetzten Bildes auf einer Anzeigevorrichtung.

12. Verfahren nach Anspruch 11, wobei die Registrierung in einem manuellen Modus verfeinert wird, in dem eine Bedienungsperson des Ultraschall-Bildgebungsgeräts visuell feststellt, dass sich die physischen Strukturen in dem extrahierten Bild mit den entsprechenden physischen Strukturen in dem zusätzlichen Echtzeitbild überlappen, wobei das auf der Anzeigeeinrichtung angezeigte zusammengesetzte Bild verwendet wird.

13. Verfahren nach Anspruch 11, wobei die Registrierung in einem automatischen Modus verfeinert wird, wobei das Verfahren ferner umfasst:

Berechnen einer Bildregistrierungsqualitäts-Metrik (IRQ-Metrik), die eine Qualität einer Ausrichtung der physischen Strukturen in dem extrahierten Bild mit den entsprechenden physischen Strukturen in dem zusätzlichen Echtzeitbild quantifiziert; und

Bereitstellen der IRQ-Metrik auf der Anzeigevorrichtung als Echtzeit-Rückmeldung an eine Bedienungsperson der Ultraschall-Bildgebungseinrichtung,

wobei die Feststellung, dass sich die physischen Strukturen (64, 66) in dem extrahierten Bild mit entsprechenden physischen Strukturen in dem zusätzlichen Echtzeitbild überlappen, auf einem optimierten Wert der berechneten IRQ-Metrik basiert.

14. Verfahren nach Anspruch 13 ferner umfassend:

Ermitteln, ob die IRQ-Metrik einen vorgegebenen Schwellenwert oder einen dynamisch bestimmten Schwellenwert erfüllt; und,

basierend auf der Ermittlung, die Anforderung, dass das mindestens eine zusätzliche Echtzeitbild räumlich dem extrahierten Bild entspricht.

15. Verfahren nach Anspruch 14, wobei der dynamisch bestimmte Schwellenwert entweder ein maximaler IRQ-Wert oder ein minimaler IRQ-Wert ist und wobei

das Verfahren ferner optional das Speichern der IRQ-Metrik und einer entsprechenden Ausrichtungspose, die die IRQ-Metrik erreicht hat, umfasst und wobei

das Verfahren ferner optional das Vergleichen einer ersten IRQ-Metrik, die in einer ersten versuchten Ausrichtungspose erhalten wurde, mit einer zweiten IRQ-Metrik, die in einer zweiten versuchten Ausrichtungspose erhalten wurde, und das Speichern der ersten versuchten Ausrichtungspose oder der zweiten versuchten Ausrichtungspose, die unter der ersten IRQ-Metrik und der zweiten IRQ-Metrik den besten IRQ-Wert erreicht hat, als Ausrichtungspose; oder

wobei die beste IRQ-Metrik einer von einer höheren IRQ-Metrik in der ersten und zweiten IRQ-Metrik, einer niedrigeren IRQ-Metrik in der ersten und zweiten IRQ-Metrik und unter der ersten und zweiten IRQ-Metrik einer IRQ-Metrik, die dem vorgegebenen Schwellenwert am nächsten ist, entspricht, und

wobei das Verfahren optional ferner das automatische Fixieren einer Ausrichtung zwischen den physischen Strukturen in dem mindestens einen zusätzlichen Echtzeitbild mit den entsprechenden physischen Strukturen in dem extrahierten Bild umfasst, wenn nach einer bestimmten Anzahl von Vergleichswiederholungen keine weitere Verbesserung der Ausrichtung möglich ist oder basierend auf einer Tendenz der IRQ-Metrik keine weitere Verbesserung der Ausrichtung erwartet wird oder nachdem die IRQ-Metrik den vorgegebenen Schwellenwert erreicht hat, und

Wählen einer zuvor gespeicherten Ausrichtungspose mit einer besten erreichten IRQ-Metrik als optimale Ausrichtungspose.

**Revendications**

1.  Système de guidage d'images chirurgicales comprenant:

    une caméra de suivi (36);
    un dispositif d'imagerie ultrasonique capable d'imager en temps réel le corps d'un patient (34), ledit dispositif d'imagerie ultrasonique étant monté sur la caméra de suivi;
    un système de suivi configuré pour recevoir des données d'images optiques de la caméra de suivi et pour déterminer la position du dispositif d'imagerie ultrasonique par rapport au corps du patient, sur la base d'une pluralité de marqueurs fiduciaires (30) attachés au corps du patient et visibles dans les données d'images optiques;
    un système de traitement d'images connecté de manière opérationnelle au dispositif d'imagerie ultrasonique et au système de suivi, le système de traitement d'images étant configuré pour:

    recevoir une image ultrasonique en temps réel (62) du patient provenant du dispositif d'imagerie ultrasonique et un ensemble de données d'images tridimensionnelles, 3D, (60) du patient provenant d'un autre dispositif d'imagerie, ladite pluralité de marqueurs fiduciaires étant également visible dans l'ensemble de données d'images tridimensionnelles;
    déterminer la position de chacun des marqueurs fiduciaires par rapport à l'ensemble de données d'images 3D;
    effectuer un enregistrement de l'image ultrasonique en temps réel avec l'ensemble de données d'images 3D, sur la base de la position du dispositif d'imagerie ultrasonique par rapport au corps du patient et de la position des marqueurs fiduciaires par rapport à l'ensemble de données d'images 3D;
    sur la base de l'enregistrement de l'image ultrasonique en temps réel avec l'ensemble de données d'images 3D, extraire de l'ensemble de données d'images 3D une image correspondant à l'espace;
    produire une image composite basée sur une superposition de l'image en temps réel avec l'image extraite de l'ensemble de données d'images 3D;
    fournir un mode dans lequel l'image ultrasonique en temps réel est libre de se déplacer par rapport à l'image extraite de l'ensemble de données d'images 3D;
    affiner l'enregistrement en recevant au moins une image ultrasonique en temps réel supplémentaire dans laquelle les structures physiques (64, 66) de l'image extraite se chevauchent avec les structures physiques correspondantes (64, 66) de l'au moins une image ultrasonique en temps réel supplémentaire; et
    fournir des moyens automatiques ou manuels pour entrer dans un mode dans lequel l'image ultrasonique en temps réel et l'image extraite de l'ensemble de données d'images 3D sont verrouillées l'une par rapport à l'autre pour empêcher tout mouvement relatif entre elles; et comprenant un dispositif d'affichage configuré pour recevoir l'image composite du système de traitement d'images et afficher l'image composite.

2.  Système selon la revendication 1, dans lequel l'enregistrement peut être affiné dans un mode manuel dans lequel un opérateur du dispositif d'imagerie ultrasonique détermine visuellement que les structures physiques dans l'image extraite se chevauchent avec les structures physiques correspondantes dans l'image supplémentaire en temps réel, en utilisant l'image composite affichée sur le dispositif d'affichage.

3.  Système selon la revendication 1, dans lequel l'enregistrement peut être affiné dans un mode automatique dans lequel le système de traitement d'images est en outre configuré pour:

    calculer une mesure de qualité d'enregistrement d'image, IRQ, qui quantifie la qualité de l'alignement des structures physiques de l'image extraite avec les structures physiques correspondantes de l'image en temps réel supplémentaire; et
    fournir sur le dispositif d'affichage la métrique IRQ en tant que retour d'information en temps réel à un opérateur du dispositif d'imagerie ultrasonique,
    la détermination du chevauchement des structures physiques (64, 66) de l'image extraite avec les structures physiques correspondantes de l'image en temps réel supplémentaire étant basée sur une valeur optimisée de la métrique IRQ calculée.

**4.** Système selon la revendication 3, dans lequel le système de traitement d'images est en outre configuré pour:

déterminer si la métrique IRQ atteint une valeur seuil prédéterminée ou une valeur seuil déterminée dynamiquement; et

en fonction de la détermination, configurer automatiquement le système de traitement d'images pour exiger qu'au moins une image en temps réel supplémentaire corresponde spatialement à l'image extraite.

**5.** Système selon la revendication 4, dans lequel

la valeur seuil déterminée dynamiquement est l'une des valeurs IRQ maximale et minimale, ou

le système de traitement d'images est configuré pour stocker la métrique IRQ et une pose d'alignement correspondante qui a atteint la métrique IRQ, ou

le système de traitement d'images est configuré pour comparer une première mesure d'IRQ obtenue lors d'une première tentative d'alignement avec une deuxième mesure d'IRQ obtenue lors d'une deuxième tentative d'alignement et pour enregistrer comme mesure d'alignement la première mesure d'alignement ou la deuxième tentative d'alignement qui a obtenu la meilleure valeur d'IRQ parmi la première mesure d'IRQ et la deuxième mesure d'IRQ.

**6.** Système selon la revendication 5, dans lequel la meilleure valeur d'IRQ correspond à l'une des valeurs suivantes: une valeur d'IRQ supérieure dans les première et deuxième valeurs d'IRQ, une valeur d'IRQ inférieure dans les première et deuxième valeurs d'IRQ, et une valeur d'IRQ parmi les première et deuxième valeurs d'IRQ qui est la plus proche de la valeur seuil prédéterminée.

éventuellement, le système de traitement d'images est configuré pour:

verrouiller automatiquement un alignement entre les structures physiques de l'au moins une image en temps réel supplémentaire et les structures physiques correspondantes de l'image extraite lorsque, après un certain nombre d'itérations de comparaison, aucune amélioration supplémentaire de l'alignement n'est possible, ou aucune amélioration supplémentaire de l'alignement n'est attendue sur la base d'une tendance de la métrique IRQ, ou après que la métrique IRQ a atteint la valeur seuil prédéterminée, et

sélectionner une position d'alignement précédemment stockée ayant une métrique IRQ optimale comme position d'alignement optimale.

**7.** Système selon la revendication 1, dans lequel le système de traitement d'images comprend un dispositif d'entrée configuré pour recevoir une première entrée d'un opérateur afin de configurer le système de traitement d'images pour permettre à l'au moins une image en temps réel supplémentaire de se mettre à jour sans correspondre spatialement à l'image extraite, et recevoir en outre une deuxième entrée de l'opérateur afin de configurer le système de traitement d'images pour exiger que l'au moins une image en temps réel supplémentaire corresponde spatialement à l'image extraite.

**8.** Système selon la revendication 3, dans lequel la métrique IRQ est calculée en effectuant une corrélation croisée bidimensionnelle entre l'image en temps réel supplémentaire et l'image extraite.

**9.** Système selon la revendication 8, dans lequel la corrélation croisée bidimensionnelle entre l'image en temps réel supplémentaire et l'image extraite est réalisée selon l'équation suivante:

$$r_{ij} = \frac{\sum_m \sum_n [f(m+i, n+j) - \bar{f}][g(m,n) - \bar{g}]}{\sqrt{\sum_m \sum_n [f(m,n) - \bar{f}]^2 \sum_m \sum_n [g(m,n) - \bar{g}]^2}}$$

où $f(m,n)$ et $g(m,n)$ sont les valeurs respectives des pixels de l'image en temps réel supplémentaire et de l'image extraite aux emplacements $m,n,$ avec $i=j=0$ pour déterminer ladite corrélation.

**10.** Système selon la revendication 1, dans lequel l'autre dispositif d'imagerie est un dispositif de tomographie assistée par ordinateur (CT), un dispositif d'imagerie par résonance magnétique (MRI) et un dispositif d'imagerie médicale tridimensionnelle.

**11.** Méthode de guidage d'images chirurgicales comprenant:

recevoir par un système de traitement d'images d'une image ultrasonique en temps réel (62) d'un patient provenant d'un dispositif d'imagerie ultrasonique, d'un ensemble de données d'images tridimensionnelles (3D) (60) du patient provenant d'un autre dispositif d'imagerie, et d'une position du dispositif d'imagerie ultrasonique par rapport au corps du patient provenant d'un système de suivi recevant des données d'images optiques d'une caméra de suivi, dans lequel une pluralité de marqueurs fiduciaires sont visibles dans l'ensemble de données d'images 3D et visibles dans les données d'images optiques;

déterminer la position de chacun des marqueurs fiduciaires par rapport à l'ensemble de données de l'image 3D;

enregistrer l'image échographique en temps réel avec l'ensemble de données d'images 3D, en fonction de la position du dispositif d'imagerie ultrasonique par rapport au corps du patient et de la position des marqueurs fiduciaires par rapport à l'ensemble de données d'images 3D;

sur la base de l'enregistrement de l'image échographique en temps réel avec l'ensemble de données d'images 3D, extraire de l'ensemble de données d'images 3D une image correspondant à l'espace;

produire par le système de traitement d'images une image composite basée sur une superposition de l'image en temps réel avec l'image extraite de l'ensemble de données d'images 3D;

fournir un mode dans lequel l'image ultrasonique en temps réel est libre de se déplacer par rapport à l'image extraite de l'ensemble de données d'images 3D;

affiner l'enregistrement en recevant au moins une image ultrasonique en temps réel supplémentaire dans laquelle les structures physiques (64, 66) de l'image extraite se superposent aux structures physiques correspondantes (64, 66) de l'image ultrasonore en temps réel supplémentaire;

fournir des moyens automatiques ou manuels pour entrer dans un mode dans lequel l'image ultrasonique en temps réel et l'image extraite de l'ensemble de données d'images 3D sont verrouillées l'une par rapport à l'autre afin d'empêcher tout mouvement relatif entre elles; et

afficher l'image composite sur un dispositif d'affichage.

**12.** Procédé selon la revendication 11, dans lequel l'enregistrement est affiné dans un mode manuel dans lequel un opérateur du dispositif d'imagerie ultrasonique détermine visuellement que les structures physiques de l'image extraite se chevauchent aux structures physiques correspondantes de l'image supplémentaire en temps réel à l'aide de l'image composite affichée sur le dispositif d'affichage.

**13.** Procédé selon la revendication 11, dans laquelle l'enregistrement est affiné dans un mode automatique dans lequel le procédé comprend en outre le calcul d'une qualité d'enregistrement d'image, IRQ, métrique, qui quantifie la qualité d'un alignement des structures physiques dans l'image extraite avec les structures physiques correspondantes dans l'image en temps réel supplémentaire; et

fournir sur le dispositif d'affichage la métrique IRQ en tant que retour d'information en temps réel à un opérateur de l'appareil d'imagerie ultrasonique,

la détermination du chevauchement des structures physiques (64, 66) de l'image extraite avec les structures physiques correspondantes de l'image en temps réel supplémentaire étant basée sur une valeur optimisée de la métrique IRQ calculée.

**14.** Procédé selon la revendication 13, comprenant en outre:

déterminer si la métrique IRQ atteint une valeur seuil prédéterminée ou une valeur seuil déterminée dynamiquement; et

en fonction de la détermination, exiger que l'au moins une image en temps réel supplémentaire corresponde spatialement à l'image extraite.

**15.** Procédé selon la revendication 14, dans lequel la valeur seuil déterminée dynamiquement est l'une des valeurs IRQ maximale et minimale, et

éventuellement, le procédé comprend en outre le stockage de la métrique IRQ et d'une pose d'alignement correspondante qui a atteint la métrique IRQ, et

éventuellement, le procédé consiste en outre à comparer une première mesure d'IRQ obtenue lors d'une première tentative d'alignement avec une deuxième mesure d'IRQ obtenue lors d'une deuxième tentative d'alignement; et à stocker en tant que mesure d'alignement la première tentative d'alignement ou la deuxième tentative d'alignement qui a atteint la meilleure valeur d'IRQ parmi la première mesure d'IRQ et la deuxième mesure d'IRQ; ou

la meilleure valeur d'IRQ correspond à une valeur d'IRQ supérieure dans les première et deuxième valeurs d'IRQ, à une valeur d'IRQ inférieure dans les première et deuxième valeurs d'IRQ et à une valeur d'IRQ parmi les première et deuxième valeurs d'IRQ qui est la plus proche de la valeur seuil prédéterminée, et

éventuellement, le procédé comprend en outre le verrouillage automatique d'un alignement entre les structures physiques de l'au moins une image en temps réel supplémentaire et les structures physiques correspondantes de l'image extraite lorsque, après un certain nombre d'itérations de comparaison, aucune amélioration supplémentaire de l'alignement n'est possible, ou aucune amélioration supplémentaire de l'alignement n'est attendue sur la base d'une tendance de la métrique IRQ, ou après que la métrique IRQ a atteint la valeur de seuil prédéterminée, et

la sélection d'une pose d'alignement précédemment stockée ayant une métrique IRQ optimale en tant que pose d'alignement optimale.

FIG. 1

FIG. 2

FIG. 3

FIG. 4

FIG. 5

FIG. 6A

FIG. 6B

## REFERENCES CITED IN THE DESCRIPTION

**Patent documents cited in the description**

- US 62426024
- US 2007083117 A
- US 648245
- US 092755
- US 092843
- US 508223
- US 524468
- US 524570
- US 689849

**Non-patent literature cited in the description**

- **WELLS et al.** Multi-Modal Volume Registration by Maximization of Mutual Information. *Medical Image Analysis*, 1996